# EUROPEAN PATENT SPECIFICATION

(11) **EP 0 435 641 B1**
(45) Date of publication and mention of the grant of the patent: **23.04.1997**
(21) Application number: 90314227.1
(22) Date of filing: 24.12.1990
(51) Int. Cl.: C07C 275/62, C07D 251/32, C07C 275/40

(54) **Polyfluoro nitrogen-containing organic compounds**
Stickstoff enthaltende organische Polyfluorverbindungen
Composés organiques polyfluorés contenant de l'azote

(30) Priority: 29.12.1989 US 459060; 29.12.1989 US 459040; 29.12.1989 US 459035
(43) Date of publication of application: 03.07.1991
(73) Proprietor: E.I. DU PONT DE NEMOURS AND COMPANY, Wilmington Delaware 19898 (US)
(72) Inventor: Kirchner, Jack Robert, Wilmington, Delaware 19803 (US)
(74) Representative: Woodcraft, David Charles

(56) References cited:
- EP-A- 0 172 717
- EP-A- 0 222 334
- EP-A- 0 253 186
- EP-A- 0 322 759
- US-A- 4 504 401
- J.H.Saunders et al, Polyurethanes, Chemistry and Technology, Part I. Chemistry, 1978, pages 76-79.
- G.Woods, Flexible Polyurethane Foams, Chemistry and Technology, 1982, pages 1-3.

## Description

The present invention relates to novel water-modified fluorochemical nitrogen-containing compounds and their use to provide oil-, water-, and soil-repellency and/or soil-release properties, and to novel processes for preparing the novel water-modified fluorochemical nitrogen-containing compounds.

U.S. 3,987,227 discloses carpets having a stain-repellent and soil-resistant coating formed by a combination of a water-insoluble fluoroaliphatic radical containing urethane adduct and a water insoluble urethane adduct free from fluoroaliphatic radicals. The urethane adducts were prepared from polyisocyanates in accordance with the process disclosed in U. S. patent 3,484,281; as is conventional with reactions involving isocyanates, anhydrous conditions were maintained.

U.S. 4,264,484 discloses treating carpets with a combination of:
(a) a water-insoluble addition polymer derived from polymerizable ethylenically unsaturated monomer; and
(b) a water-insoluble fluoroaliphatic radical and aliphatic chlorine-containing carboxylic or carbamic ester.

U.S. 4,340,749 discloses treating carpets to render them soil-resistant and stain-repellent with a carboxylic or carbamic ester of a fluoro-aliphatic radical- and aliphatic chlorine-containing alcohol.

U.S. 4,401,780 discloses treating textiles with a fluorochemical composition comprising a mixture of:
(a) a water-insoluble fluoroaliphatic radical and aliphatic chlorine-containing ester;
(b) a water-insoluble fluoroaliphatic radical-containing polymer; and
(c) water insoluble fluoroaliphatic radical-containing compound selected from carbonylimino compounds and imine compounds.

U.S. patent 4,504,401 discloses polyfluoroalkyl compounds, said to be useful as stain proofing agents, which are obtained by reacting a polyfluoroalkyl-containing alcohol with a polyfunctional isocyanate compound containing at least three isocyanate groups. In addition to the polyfluoroalkyl-containing compounds, nonfluorinated compounds may be reacted with the polyisocyanate, most notably stearyl alcohol. Reaction of the polyisocyanate with the polyfluoroalkyl-containing compound and the nonfluorinated compounds are carried out sequentially. Substantially anhydrous conditions are used for those reactions, because the presence of water is indicated to be disadvantageous to the isocyanate group.

U.S. patent 4,668,406 discloses fluorochemical biurets which have one or more monovalent fluoroaliphatic radicals and one or more biuret moieties, and can contain organoamino or acid moieties. The flurochemical biurets are said to be useful for the treatment of fibrous substrates to impart oil- and water-repellency. Various reaction schemes are disclosed, none of which discloses the use of water as a reactant; in the experiments described in the EXAMPLES, the reactions are conducted under nitrogen.

Br, 1,241,505 discloses tertiary amine salts of fluorocarbamates which are useful as oil-repellents for treating fabrics.

US Patent No. 3,201,372 discloses reacting a stoichiometric excess of a polyisocyanate with a polyol containing no fluorine, then reacting the resulting free isocyanate-containing prepolymer with water so as to produce a polymeric foam.

EP-A-0172717 describes a polyfluoro organic compound for use in treating fibres or fabrics to impart water, oil or soil release properties. The polyfluoro compound is prepared by reacting a tris (isocyanato)alkane biuret with a fluoroalcohol and then with a non-fluorine containing organic compound containing active hydrogen atoms, such as a monoalcohol, diol or triol. There is no disclosure of reacting the reaction product of the biuret and fluoroalcohol with water.

The present invention relates to novel fluorochemical nitrogen-containing compounds which when applied to fibers and fabrics provide durable water-, oil, and soil-repellent and/or soil-release properties to the fibrous substrates. It relates also to substrates treated with the novel fluorochemical compounds of the invention. It relates further to processes for imparting water-, oil-, and soil-repellency and/or soil-release properties to fibrous substrates wherein the novel compounds of the invention are applied to the substrates. It relates in addition to novel processes for preparing the novel fluorochemical compounds of the invention.

According to one aspect of the present invention there is provided a polyfluoro organic compound comprising a compound having at least one urea linkage which compound is the reaction product of (1) an excess of at least one polyisocyanate, with (2) at least one fluorochemical compound which contains per molecule (a) a single functional group having one or more Zerewitinoff hydrogen atoms and (b) at least two carbon atoms each of which contains at least two fluorine atoms, and then with (3) water, the water being used in an in an amount sufficient to react with all residual isocyanate groups, said residual groups constituting from 5% to 60% of the isocyanate groups in said polyisocyanate, wherein said polyisocyanate contains at least three isocyanate groups.

In a preferred embodiment, the amount of water is sufficient to react with 10% to 35% of the isocyanate groups in the polyisocyanate, and more preferably, between 15% and 30%.

A wide variety of fluorochemical compounds which contain a single functional group can be used so long as each fluorochemical compound contains at least two carbon atoms and each carbon atom contains at least two fluorine atoms. For example, the fluorochemical compound can be represented by the formula:

R^{f}-Rₖ-X-H

wherein
R^{f} is a monovalent aliphatic group containing at least two carbon atoms each of which contains at least two fluorine atoms;
R is a divalent organic radical;
k is 0 or 1; and
X is -O-, -S-, or -N(R¹)-² in which R¹ is H, alkyl containing 1 to 6 carbon atoms or a R^{f}-Rₖ- group.
In a more specific embodiment, the fluorochemical compound which contains a single functional group can be represented by the formula:

R^{f}-Rₖ-R²-X-H

wherein
- R^{f} and k: are as defined above;
- R: is the divalent radical -CₘH₂ₘSO-, -CₘH₂ₘSO₂-, -SO₂N(R³)-, or -CON(R³)- in which m is 1 to 22 and R³ is H or alkyl of 1 to 6 carbon atoms;
- R²: is the divalent linear hydrocarbon radical -CₙH₂ₙ- which can be optionally end-capped by 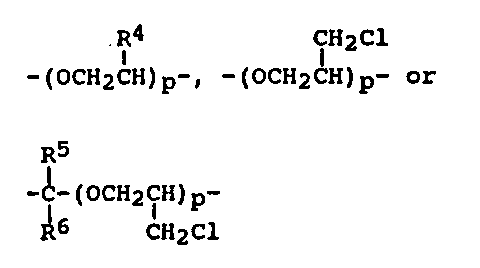 in which n is 0 to 12, p is 1 to 50, and R⁴, R⁵ and R⁶ are the same or different H or alkyl containing 1 to 6 carbon atoms; and
- X: is -O-, -S-, or -N(R⁷)- in which R⁷ is H, alkyl containing 1 to 6 carbon atoms or a R^{f}-Rₖ-R²- group.
More particularly, R^{f} is a fully-fluorinated straight or branched aliphatic radical of 3 to 20 carbon atoms which can be interrupted by oxygen atoms.

In a preferred embodiment, the fluorochemical compound which contains a single functional group can be represented by the formula:

R^{f}-(CH₂)_{q}-X-H

wherein
- X: is -O-, -S-, or -N(R⁷)- in which R⁷ is H, alkyl containing 1 to 6 carbon atoms or a R^{f}-Rₖ-R²- group.
- R^{f}: is the perfluoroalkyl group CF₃CF₂(CF₂)ᵣ in which r is 2 to 18; and
- q: is 1, 2 or 3.
In a more particular embodiment, R^{f} is a mixture of said perfluoroalkyl groups, CF₃CF₂(CF₂)ᵣ; and r is 2, 4, 6, 8, 10, 12, 14, 16, and 18. In a preferred embodiment, r is predominantly 4, 6 and 8; e.g. see Example 1. In another preferred embodiment, r is predominantly 6 and 8; e.g. see Example 44. The former preferred embodiment is more readily available commercially and is therefore less expensive, while the latter may provide improved properties.

Representative fluoroaliphatic alcohols that can be used for the purposes of this invention are:
C_{S}F_{2S+1}(CH₂)ₜOH
(CF₃)₂CFO(CF₂CF₂)ᵤCH₂CH₂OH
C_{S}F_{2S+1}CON(R⁸)(CH₂)ₜOH
C_{S}F_{2S+1}SO₂N(R⁸)(CH₂)ₜOH 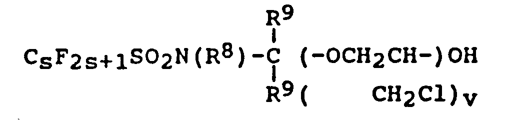 wherein
s is 3 to 14;
t is 1 to 12;
u is 1 to 5;
   each of R⁸ & R⁹ is H or alkyl containing 1 to 6 carbon atoms

In another embodiment, the fluorochemical compound which contains a single functional group can be represented by the formula: H(CF₂CF₂)_{w}CH₂OH wherein w is 1-10. The latter fluorochemical compound is a known fluorochemical compound which can be prepared by reacting tetrafluoroethylene with methanol. Yet another such compound is 1,1,1,2,2,2-hexafluoro-isopropanol having the formula: CF₃(CF₃)CHOH.

In yet another embodiment of the invention, a nonfluorinated organic compound which contains a single functional group can be used in conjunction with one or more of said fluorochemical compounds. Usually between 1% and 60% of the isocyanate groups of the polyisocyanate are reacted with at least one such nonfluorinated compound. For example, said non-fluorinated compound can be represented by the formula:

R¹⁰-R¹¹ₖ-YH

wherein
- R¹⁰: is a C₁-C₁₈ alkyl, a C₁-C₁₈ omega-alkenyl radical or a C₁-C₁₈ omega-alkenoyl;
- R¹¹: is 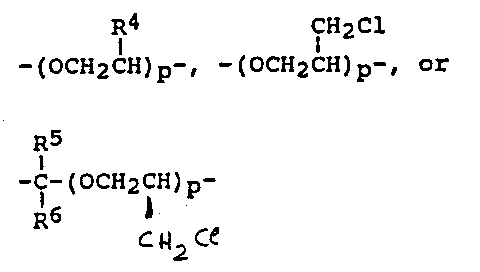 in which R⁴, R⁵ and R⁶ are the same or different H or alkyl radical containing 1 to 6 carbon atoms and p is 1 to 50;
- Y: is -O-, -S-, or -N(R⁷)- in which R⁷ is H or alkyl containing 1 to 6 carbon atoms; and
- k and p: are as defined above.
For example, the nonfluorinated compound can be an alkanol or a monoalkyl or monoalkenyl ether or ester of a polyoxyalkylene glycol. Particular examples of such compounds include stearyl alcohol, the monomethyl ether of polyoxethylene glycol, the mono-allyl or -methallyl ether of polyoxethylene glycol, the mono-methacrylic or acrylic acid ester of polyoxethylene glycol, and the like.

Any polyisocyanate having three or more isocyanate groups can be used for the purposes of this invention. For example, one can use hexamethylene diisocyanate homopolymers having the formula: 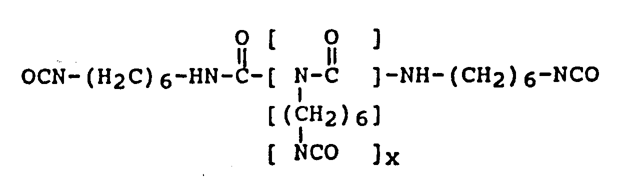 wherein x is an integer equal to or greater than 1, preferably between 1 and 8. Because of their commercial availability, mixtures of such hexamethylene diisocyanate homopolymers are preferred for purposes of this invention. Also of interest are hydrocarbon diisocyanate-derived isocyanurate trimers which can be represented by the formula: 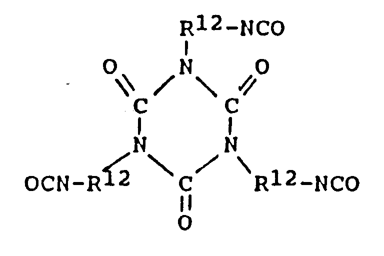 wherein R¹² is a divalent hydrocarbon group, preferably aliphatic, alicyclic, aromatic or arylaliphatic. For example, R¹² can be hexamethylene, toluene or cyclohexylene, preferably the former. Other polyisocyanates useful for the purposes of this invention are those obtained by reacting three mols of toluene diisocyanate with 1,1,1-tris-(hydroxymethyl)-ethane or 1,1,1-tris-(hydroxymethyl)-propane. The isocyanurate trimer of toluene diisocyanate and that of 3-isocyanatomethyl-3,4,4-trimethylcyclohexyl isocyanate are other examples of polyisocyanates useful for the purposes of this invention, as is methine-tris-(phenylisocyanate). Also useful for the purposes of this invention is the polyisocyanate having the formula:

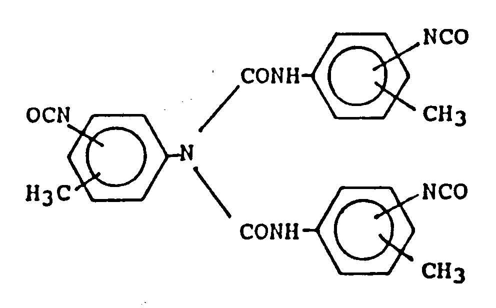

The polyfluoro organic compounds of the invention are prepared by reacting: (1) at least one polyisocyanate or mixture of polyisocyanates which contains at least three isocyanate groups per molecule with (2) at least one fluorochemical compound which contains per molecule (a) a single functional group having one or more Zerewitinoff hydrogen atoms and (b) at least two carbon atoms each of which contains at least two fluorine atoms. Thereafter the remaining isocyanate groups are reacted with water to form one or more urea linkages. Usually between 40% and 95% of the isocyanate groups will have been reacted before water is reacted with the polyisocyanate. In other words, the amount of water generally is sufficient to react with from 5% to 60% of the isocyanate groups in the polyisocyanate. Preferably, between 60% and 90% of the isocyanate groups have been reacted before water is reacted with the polyisocyanate, and most preferably between 70% and 85% of the isocyanate groups have been reacted prior to reaction of water with the polyisocyanate. Thus, in a preferred embodiment the amount of water is sufficient to react with 10% to 35% of the isocyanate groups, most preferably between 15% and 30%.

In one embodiment, water-modified fluorochemical carbamates have been prepared by the sequential catalyzed reaction of Desmodur N-100, Desmodur N-3200 or Desmodur N-3300, or mixtures thereof, with a stoichiometric deficiency of a perfluoroalkyl compound containing one functional group, and then with water. Desmodur N-100 and Desmodur N-3200 are hexamethylene diisocyanate homopolymers commercially available from Mobay Corporation. Both presumably are prepared by the process described in U.S. Patent No. 3,124,605 and presumably to give mixtures of the mono, bis, tris, tetra and higher order derivatives which can be represented by the general formula: 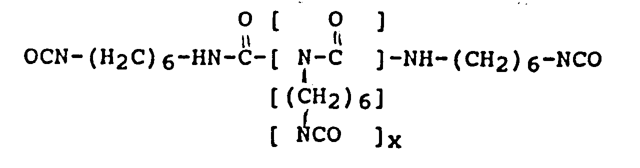 wherein x is an integer equal to or greater than 1, preferably between 1 and 8.

| Typical Properties | Ave. Eq. Wt. | NCO Content, % |
|---|---|---|
| Desmodur N-100 | 191 | 22.0 |
| Desmodur N-3200 | 181 | 23.2 |

The typical NCO content of Desmodur N-100 approximates that listed for a SRI International Report (Isocyanates No. 1D, July, 1983, Page 279) hexamethylene diisocyanate homopolymer with the following composition:

| Product Composition | Wt. % |
|---|---|
| Hexamethylene diisocyanate | 0.1 |
| Monobiuret | 44.5 |
| Bisbiuret | 17.4 |
| Trisbiuret | 9.5 |
| Tetrabiuret | 5.4 |
| Higher Mol. Wt. Derivatives | 23.1 |
| NCO Content | 21.8 |

Based on its average equivalent weight and NCO content, the comparative bis, tris, tetra, etc., content of Desmodur N-3200 should be less than that of the N-100 product. Desmodur N-3300 is a hexamethylene diisocyanate-derived isocyanurate trimer which can be represented by the formula:

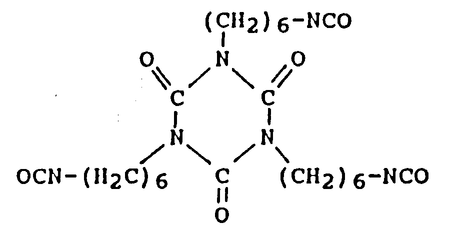

The water-modified fluorochemical carbamates are typically prepared by first charging the polyisocyanate, the perfluoroalkyl compound and a dry organic solvent such as methyl isobutyl ketone (MIBK) to a reaction vessel. The order of reagent addition is not critical. The specific weight of aliphatic polyisocyanate and perfluoroalkyl compounds charged is based on their equivalent weights and on the working capacity of the reaction vessel and is adjusted so that all Zerewitinoff active hydrogens charged will react with some desired value between 40% and 95% of the total NCO group charge. The weight of dry solvent is typically 15%-30% of the total charge weight. The charge is agitated under nitrogen and heated to 40°-70°C. A catalyst, typically dibutyltindilaurate per se, or as a solution in methylisobutylketone (MIBK), is added in an amount which depends on the charge, but is usually small, e.g. 1 to 2 parts per 10,000 parts of the polyisocyanate. After the resultant exotherm, the mixture is agitated at a temperature between 65° and 105°C for 2-20 hours from the time of the catalyst addition, and then, after its temperature is adjusted to between 55° and 90°C, is treated with water per se or with wet MIBK for an additional 1 to 20 hours. The resultant product can be stored and/or used as prepared or after further solvent dilution or converted by standard technology to a emulsion or dispersion is surfactant-stabilized; in others, a stable emulsion or dispersion can be prepared without the use of a surfactant, e.g. that of Example 53.

Suitable substrates for the application of the products of this invention are films, fibers, yarns, fabrics, carpeting, and other articles made from filaments, fibers, or yarns derived from natural, modified natural, or synthetic polymeric materials or from blends of these other fibrous materials and other porous materials which will absorb and transport low surface tension liquids either on their surfaces on in their interstices by capillary action. Specific representative examples are cotton, silk, regenerated cellulose, nylon, fiber-forming linear polyesters, fiber-forming polyacrylonitrile,cellulose nitrate, cellulose acetate, ethyl cellulose, paper, fiberglass, wood pressed or otherwise hardened wood composites, metals, unglazed porcelain, porous concrete and the like. Dyed and undyed cotton sateen, poplin, broadcloth, jean cloth, gabardine and the like are especially adaptable for treatment with the compositions of this invention to provide products having a high repellency to oil and water and which are also relatively unaffected by the action of heat, air and light. Materials rendered oil and water-repellent by the products of this invention retain a high portion of the original repellency after laundering and dry cleaning. The novel compounds of this invention impart oil-, water- and soil-repellency and/or soil-release properties to fibrous and non-fibrous substrates.

Two types of substrates are of particular interest in accordance with the present invention. One of those is carpeting, particularly nylon carpeting, to which novel compounds of the present invention are applied so as to impart oil-, water- and soil-repellency. The other class of substrates to which it is particularly advantageous to apply the compounds of the present invention so as to impart soil-release properties includes those prepared from polyamide fibers (such as nylon), cotton and blends of polyester and cotton, particularly such substrates being used in tablecloths, washable uniforms and the like. Of particular interest are polyamide carpeting, e.g. nylon carpeting, to which a preferred embodiment illustrated by Example 26 is applied so as to impart oil-, water-, and soil-repellency. In another preferred embodiment, the novel compound exemplified by Example 53 is applied to textile fabrics so as to impart soil-release properties.

The compounds of the present invention can be applied to suitable substrates by a variety of customary procedures. For the carpeting end-use, one can apply them by use of the conventional beck dying procedure, continuous dying procedure or thread-line application. For application to washable apparel fabrics, the compounds of the present invention can be applied, for example, from an aqueous dispersion or an organic solvent solution by brushing, dipping, spraying, padding, roll-coating, foaming or the like. The compounds of this invention can be applied to the substrate as such or in combination with other textiles or fluoro-finishes, processing aids, lubricants, anti-stains, etc. The compounds can also be blended with other agents which have oil/water repellency and soil release properties and applied to fibers or fabrics. They can be applied to dyed and undyed carpeting and other textile substrates.

The use of a stoichiometric excess of a polyisocyanate assures complete reaction of the fluorinated and nonfluorinated organic compounds; that coupled with subsequent reaction with water provides products of this invention which possess enhanced properties when compared to those of the prior art, particularly when used to treat carpeting, as well as washable fabrics such as table clothes, uniforms and the like. In addition those aspects of the invention eliminate any need to remove any unreacted organic compound. It thus provides a substantial process advantage; it also provides greater product purity and uniformity.

In the Examples that follow, the test described below were used.

### Test No. 1 - Wash Stability

Nylon 66 knit fabric is padded with a FREON-113/acetone (1/6 parts by weight) solution of the fluorochemical so as to obtain a uniform coverage of about 700 ppm fluorine (on the weight of the fiber). The air dried fabric sample is annealed for two minutes in a circulating air oven at 394°F, a swatch then taken for fluorine analysis and the remainder subjected to five standard home washing cycles and reanalyzed for flourine content and, for some samples, oil/water repellency. The home washes are carried out in a "Kenmore" washing machine at 40°C, employing 28 grams of "Tide" detergent per washload, followed by drying for 40 minutes in an automatic dryer at medium setting.

### Test No. 2 - Oil/Water Repellency

Beginning with the lowest numbered test liquid (Repellency Rating No. 1), one drop (approximately 5 mm diameter or 0.05-ml volume) is placed on each of three locations at least 5 mm apart. The drops are observed for 10 seconds for the water-repellency test, 30 seconds for the oil-repellency test. If, at the end of those periods of time, two of the three drops are still spherical to hemispherical in shape with no wicking around the drops, three drops of the next higher numbered test liquid are placed on adjacent sites and observed again for the specified periods of time. The procedure is continued until one of the test liquids results in two of the three drops failing to remain spherical or hemispherical, or wetting or wicking occurs. The oil-repellency rating and the water-repellency rating of the yarn, fabric or carpet each is the highest numbered test liquid for which two of three drops remain spherical or hemispherical with no wicking for the specified time.

### STANDARD WATER TEST LIQUIDS

| Water-Repellency Rating Number | Composition (Volume %) | |
|---|---|---|
| | Isopropanol (Reagent Grade) | Distilled H₂O |
| 1 | 2 | 98 |
| 2 | 5 | 95 |
| 3 | 10 | 90 |
| 4 | 20 | 80 |
| 5 | 30 | 70 |

Unless otherwise indicated in the Examples that follow, the fluorinated reactant is the above-described perfluoroalkylethyl alcohol mixture (FA) of the formula: F(CF₂)_{y}CH₂CH₂OH and/or perfluoroalkylpropyl amine mixture of the formula: F(CF₂)_{y}CH₂CH₂CH₂NH₂, wherein y in each formula is predominantly 6, 8 and 10. In a typical mixture of such fluoroalcohols or amines, the compounds will have the following approximate composition in relation to their F(CF₂)_{y} radicals:
- 0% to 3%: wherein y = 4,
- 27% to 37%: wherein y = 6,
- 28% to 32%: wherein y = 8,
- 14% to 20%: wherein y = 10,
- 8% to 13%: wherein y = 12,
- 3% to 6%: wherein y = 14,
- 0% to 2%: wherein y = 16,
- 0% to 1%: wherein y = 18, and
- 0% to 1%: wherein y = 20.
Oil and water repellencies are given in Tables 1 and 2 under the heading "O/W". In some of the Examples, the above-described two-step procedure was used in which a polyisocyanate containing at least three NCO groups was reacted with a perfluoroalkyl alcohol and/or amine to obtain an intermediate product in which the perfluoroalkyl and isocyanate group-containing moieties are linked by a urethane or urea group, respectively. In other Examples, a non-fluorinated organic compound which contains a single functional group was reacted with the polyisocyanate along with the fluoroalcohol and/or fluoroamine. The subsequent reaction of water with the residual NCO groups is presumed to predominantly yield urea group linked products by one or both of the following reaction pathways: 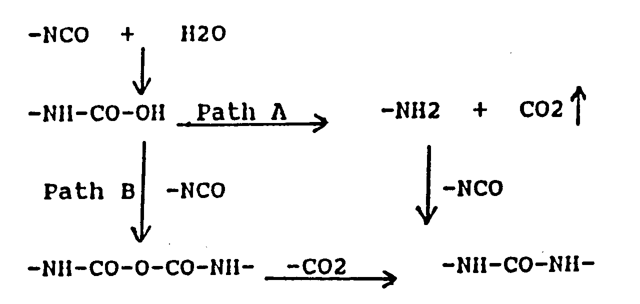 Water reacting by either of the two pathways acts as a dual functional Zerewitinoff active hydrogen compound. It is convenient to describe the amount of water added to a synthesis mixture in terms of the amount of Zerewitinoff active hydrogens added per number of residual NCO groups, ie as a ratio. The theoretical water ratio required to satisfy the stoichiometry of the two pathways is 1.0.

### Example 1

A hexamethylene diisocyanate homopolymer (Desmodur N-100) containing 22.05% NCO groups by di-n-butylamine titration method analysis, a perfluoroalkylethyl alcohol mixture (FA) in an amount sufficient to react with 95.1% of the NCO group charge, and dry MIBK in an amount equal to 24.1% of the total charge weight were added to a reaction vessel, agitated under nitrogen and heated to 70°C, whereupon a catalytic amount of dibutyltindilaurate was added. After the resultant exotherm, the reaction mixture was agitated at about 80°C for 2 hours from the time of the catalyst addition, and then treated with wet MIBK in an amount equal to a water ratio of 8.96. The diluted mixture was agitated at about 65°C for an additional 2.5 hours. When tested for oil- and water- repellency (O/W) pursuant to Tests 1 and 2, the product gave ratings of 7/6.

### Examples 2-5

The procedure of Example 1 was repeated with the modifications set forth in Table 1.

**Table 1**

| Ex. | Step One | | | Step Two | | %F Retained | O/W |
|---|---|---|---|---|---|---|---|
| | Rx %* | MIBK % | Cat °C | Water Ratio | Time, Hours | | |
| 2 | 89.3 | 24.9 | 65 | 3.84 | 16 | 64 | 6/6 |
| 3 | 85.0 | 23.5 | 70 | 2.74 | 2.5 | 67 | 7/7 |
| 4 | 79.2 | 24.3 | 60 | 1.85 | 16 | 73 | 7/6 |
| 5 | 69.3 | 24.6 | 62 | 1.13 | 16 | 77 | 6/7 |

| | | | | | | | |
|---|---|---|---|---|---|---|---|
| * Percentage of available -NCO groups reacted with FA. | | | | | | | |

### Example 6

A hexamethylene diisocyanate homopolymer (Desmodur N-3200) containing 22.60% NCO groups by di-N-butylamine titration method analysis, a perfluoroalkylethyl alcohol mixture (FA) in an amount sufficient to react with 59.9% of the NCO group charge, and dry MIBK in an amount equal to 22.4% of the total charge weight were added to a reaction vessel, agitated under nitrogen and heated to 69°C, whereupon a catalytic amount of dibutyltindilaurate was added. After the resultant exotherm, the reaction mixture was agitated at about 80°C for 2 hours from the time of the catalyst addition, then cooled to 65°C and treated with wet MIBK in an amount equal to a water ratio of 1.00. The diluted reaction mixture was agitated at about 65°C for an additional 17.5 hours. The product had an O/W rating of 6/6.

### Examples 7-10

The procedure of Example 6 was repeated with the modifications set forth in Table 2.

**Table 2**

| Ex. | Step One | | | Step Two | | %F Retained | O/W |
|---|---|---|---|---|---|---|---|
| | Rx % | MIBK % | Cat °C | Water Ratio | Time, Hours | | |
| 7 | 66.5 | 22.6 | 63 | 1.00 | 17 | 81 | 7/7 |
| 8 | 73.7 | 22.9 | 65 | 1.00 | 17 | 70 | 7/7 |
| 9 | 80.0 | 23.0 | 68 | 1.00 | 18 | 71 | 7/7 |
| 10 | 89.6 | 23.2 | 67 | 1.01 | 18 | 64 | 7/6 |

### Example 11

A hexamethylene derived isocyanurate trimer (Desmodur N-3300) containing 21.65% NCO groups by di-N-butylamine titration method analysis, a perfluoroalkylethyl alcohol mixture (FA) in an amount sufficient to react with 59.9% of the NCO group charge and dry MIBK in an amount equal to 22.1% of the total charge weight were added to a reaction vessel, agitated under nitrogen and heated to 59°C, whereupon a catalytic amount of dibutyltindilaurate was added. After the resultant exotherm, the reaction mixture was agitated at about 80°C for 4.25 hours from the time of the catalyst addition, and then treated with wet MIBK in an amount equal to a water ratio of 1.58. The diluted mixture was agitated at about 65°C for an additional 17 hours.

### Examples 12-14

The procedure of Example 6 was repeated with the modifications set forth in Table 3.

**Table 3**

| Ex. | Step One | | | Step Two | |
|---|---|---|---|---|---|
| | Rx % | MIBK % | Cat °C | Water Ratio | Time Hours |
| 12 | 70.0 | 22.0 | 62 | 2.46 | 17 |
| 13 | 79.9 | 22.3 | 60 | 2.04 | 17 |
| 14 | 89.2 | 22.7 | 62 | 4.08 | 17 |

### Example 15

A hexamethylene diisocyanate homopolymer (Desmodur N-3200) and a hexamethylene derived isocyanurate (Desmodur N-3300) containing respectively 22.51% and 21.65% NCO groups by di-N-butylamine titration method analysis, were charged to a reaction vessel in relative amounts that the biuret mixture contributed 74.9% of the total NCO group charge, along with a perfluroalkylethyl alcohol (FA) in an amount sufficient to react with 69.9% of the total NCO group charge, and dry MIBK in an amount equal to 22.2% of the total charge weight. The mixture was agitated under nitrogen and heated to 69°C, whereupon a catalytic amount of dibutyltindilaurate was added. After the resultant exotherm, the reaction mixture was agitated at about 80°C for 2 hours from the time of the catalyst addition and then treated with wet MIBK in an amount equal to a water ratio of 2.44. The diluted reaction mixture was agitated at about 65°C for 4 additional hours.

### Example 16

The procedure of Example 15 was repeated except the biuret mixture contributed 87.6% of the total NCO group charge.

### Example 17

A hexamethylene diisocyanate homopolymer (Desmodur N-3200) containing 22.54% NCO groups by di-N-butylamine titration method analysis, a perfluoroalkylethyl alcohol mixture (FA) in an amount sufficient to react with 59.9% of the total NCO group charge, a perfluoroalkylpropyl amine mixture in an amount sufficient to react with 10.0% of the total NCO group charge and dry MIBK in an amount equal to 21.9% of the total charge weight were added to a reaction vessel, agitated under nitrogen and heated to 57°C, whereupon a catalytic amount of dibutyltindilaurate was added. After the resultant exotherm, the reaction mixture was agitated at about 80°C for 3.25 hours from the time of the catalyst addition, and then treated with wet MIBK in an amount equal to a water ratio of 1.25. The diluted reaction mixture was agitated at about 65°C for an additional 17.5 hours.

### Example 18

The product was prepared by the procedure of Example 17 except the perfluoroalkylethyl alcohol mixture (FA) and the perfluoroalkylpropyl amine mixture were added in amounts sufficient to react with 50.0% and 20.0%, respectively, of the total NCO group charge.

### Example 19

A hexamethylene diisocyanate homopolymer (Desmodur N-3200) containing 22.66% NCO groups by di-N-butylamine titration method analysis, a perfluoroalkylethyl alcohol mixture (FA) in an amount sufficient to react with 70.1% of the NCO group charge, and dry MIBK in an amount equal to 22.9% of the total charge weight were added to a reaction vessel, agitated under nitrogen and heated to 60°C, whereupon a catalytic amount of dibutyltindilaurate was added. After the resultant exotherm, the reaction mixture was agitated at about 80°C for three hours from the time of the catalyst addition, then cooled to 61°C and treated with water in an amount equal to a water ratio of 38.62. The treated mixture was agitated at about 65°C for an additional 17 hours.

### Example 20

The product was prepared from a hexamethylene diisocyanate homopolymer (Desmodur N-3200) containing 22.60% NCO groups by di-N-butylamine titration method analysis by the procedure of Example 19, with MIBK at 22.8%, a temperature of 57°C at the time that the catalyst was added, a water ratio of 1.00, and a reaction period of 18 hours in the second step.

### Example 21

A hexamethylene diisocyanate homopolymer (Desmodur N-3200) containing 22.53% NCO groups by di-N-butylamine titration method analysis, a perfluoroalkylethyl alcohol mixture (FA) in an amount sufficient to react with 69.9% of the NCO group charge, and dry MIBK in an amount equal to 22.2% of the total charge weight were added to a reaction vessel, agitated under nitrogen and heated to 65°C, whereupon a catalytic amount of dibutyltindilaurate was added. After the resultant exotherm, the reaction mixture was agitated at about 80°C for two hours from the time of the catalyst addition, and then treated with three approximately equal portions of water at 1/2 hour intervals in a total amount corresponding to a water ratio of 1.43. The treated mixture was agitated at about 80°C for four hours from the first addition of water.

### Example 22

A hexamethylene diisocyanate homopolymer (Desmodur N-3200) in dry methylisobutylketone solution containing 13.69% NCO groups by di-N-butylamine titration method analysis and a perfluoroalkylethyl alcohol mixture (FA) in an amount sufficient to react with 50.0% of the NCO group charge were added to a reaction vessel, agitated under nitrogen and heated to 62°C whereupon a catalytic amount of dibutyltin-dilaurate was added. After the resultant exotherm, the reaction mixture was agitated at about 80°C for two hours from the time of the catalyst addition, and then treated with wet MIBK in an amount equal to a water ratio of 0.99. The diluted mixture was agitated at about 65°C for an additional 18 hours. The product gave an O/W rating of 5/8.

### Examples 23-27

The procedure of Example 22 was repeated with the modifications set forth in Table 4.

**Table 4**

| Ex. No. | Step One | | Step Two | | O/W |
|---|---|---|---|---|---|
| | Rx, % | Cat., °C | Water Ratio | Ratio Hours | |
| 23 | 54.7 | 62 | 1.16 | 17 | 5/7 |
| 24 | 60.0 | 62 | 1.43 | 17 | 5/7 |
| 25 | 70.0 | 62 | 2.21 | 16 | 6/8 |
| 26 | 75.0 | 62 | 2.83 | 16 | 6/8 |
| 27 | 90.0 | 60 | 1.11 | 17 | 5/5 |

### Example 28

A hexamethylene diisocyanate homopolymer (Desmodur N-3200) in dry methylisobutylketone solution containing 13.69% NCO groups by di-N-butylamine titration method analysis, a perfluoroalkylethyl alcohol mixture (FA) in an amount sufficient to react with 70.0% of the NCO group charge and 1-octadecanol (stearyl alcohol) in an amount sufficient to react with 20.0% of the NCO group charge were added to a reaction vessel, agitated under nitrogen and heated to 62°C, whereupon a catalytic amount of dibutyltindilaurate was added. After the resultant exotherm, the reaction mixture was agitated at about 80°C for 2 hours from the time of the catalyst addition and then treated with wet MIBK in an amount equal to a water ratio of 7.41. The diluted reaction mixture was agitated at about 65°C for an additional 17 hours.

### Example 29

A hexamethylene diisocyanate homopolymer (Desmodur N-3200) in dry MIBK solution containing 13.66% NCO groups by di-N-butylamine titration method analysis, a perfluoroalkylethyl alcohol mixture (FA) in an amount sufficient to react with 75.0% of the NCO group charge and a monomethyl ether of a poly-(oxyethylene) glycol, 750 average molecular weight (MPEG 750), in an amount sufficient to react with 20.0% of the NCO group charge were added to a reaction vessel, agitated under nitrogen and heated to 62°C, whereupon a catalytic amount of dibutyltindilaurate was added. After the resultant exotherm, the reaction mixture was agitated at about 80°C for 2 hours from the time of the catalyst addition and then treated with wet MIBK in an amount equal to a water ratio of 21.09. The diluted reaction mixture was agitated at about 65°C for an additional 2 hours.

### Example 30

The procedure of Example 29 was repeated except that the amount of MPEG 750 was sufficient to react with 10% of the NCO group charge, and the water ratio was 6.02.

### Example 31

The procedure of Example 29 was repeated except that the amount of MPEG 750 was sufficient to react with 5.0% of the NCO group charge, and the water ratio was 4.11.

### Example 32

The procedure of Example 29 was repeated except that the amount of FA was sufficient to react with 73.0% of the NCO group charge; the amount of MPEG 750 was sufficient to react with 2.0% of the NCO group charge, and the water ratio was 3.05.

### Example 33

A hexamethylene (Desmodur N-3200) in dry MIBK solution containing 13.52% NCO groups by di-N-butylamine titration method analysis and a perfluoroalkylethyl alcohol mixture (FA) in an amount sufficient to react with 40.0% of the NCO group charge were added to a reaction vessel, agitated under nitrogen and heated to 60°C, whereupon a catalytic amount of dibutyltindilaurate was added. After the resultant exotherm, the reaction mixture was agitated at about 80°C for two hours from the time of the catalyst addition and then treated with wet MIBK in an amount equal to a water ratio of 1.05:1. The diluted reaction mixture was agitated at about 65°C for an additional two hours.

### Example 34

Example 33 was repeated except that the amount of FA was sufficient to react with 45.0% of the NCO group charge; the temperature was 62°C, and the water ratio was 1.07.

### Example 35

A hexamethylene diisocyanate homopolymer (Desmodur N-3200) in dry MIBK solution containing 13.66% NCO groups by di-N-butylamine titration method analysis and a perfluoroalkylethyl alcohol mixture (FA) in an amount sufficient to react with 95.0% of the NCO group charge were added to a reaction vessel, agitated under nitrogen and heated to 60°C whereupon a catalytic amount of dibutyltindilaurate was added. After the resultant exotherm, the reaction mixture was agitated at about 80°C for two hours from the time of the catalyst addition, and then treated with wet MIBK in an amount equal to a water ratio of 18.78. The diluted reaction mixture was agitated at about 85°C for an additional two hours.

### Example 36

A mixture of hexamethylene diisocyanate homopolymer (Desmodur N-3200) in dry MIBK (containing 13.52% NCO groups by di-N-butylamine titration method analysis) and 2,2,2,3,3,3-hexafluoroisopropanol in an amount sufficient to react with 80.0% of the NCO group charge were added to a reaction vessel and agitated under nitrogen. After the resultant exotherm, the reaction mixture was heated to 63°C, whereupon a catalytic amount of dibutyltindilaurate was added. The reaction mixture was heated further to and held at about 80°C for two hours from the time of the catalyst addition, and then treated with wet MIBK in an amount equal to a water ratio of 1.58. The diluted reaction mixture was agitated at about 65°C for an additional two hours.

### Example 37

A hexamethylene diisocyanate homopolymer (Desmodur N-3200) in dry MIBK solution containing 13.52% NCO groups by di-N-butylamine titration method analysis and 2,2,3,3-tetrafluoro-1-propanol in an amount sufficient to react with 75.0% of the NCO group charge were added to a reaction vessel, agitated under nitrogen and heated to 60°C, whereupon a catalytic amount of dibutyltindilaurate was added. After the resultant exotherm, the reaction mixture was agitated at about 80°C for two hours from the time of the catalyst addition, and then treated with wet MIBK in an amount equal to a water ratio of 1:1. The diluted reaction mixture was agitated at about 65°C for an additional two hours.

### Example 38

A hexamethylene diisocyanate homopolymer (Desmodur N-3200) containing 22.54% NCO groups by di-N-butylamine titration method analysis, N-methyl-N-ethanolperfluorooctane sulfonamide in an amount sufficient to react with 70.0% of the total NCO group charge, and dry MIBK in an amount equal to 22.3% of the total charge weight were added to a reaction vessel, agitated under nitrogen and heated to 60°C for three hours from the time of the catalyst addition, and then treated with wet MIBK in an amount equal to a water ratio of 1.36. The diluted mixture was agitated at about 65°C for an additional 17.5 hours.

### Example 39

A hexamethylene diisocyanate homopolymer (Desmodur N-3200) containing 22.54% NCO groups by di-N-butylamine titration method analysis, N,N-bis-(perfluorooctylethyl)-amine in an amount sufficient to react with 69.9% of the total NCO group charge, and dry MIBK in an amount equal to 22.5% of the total charge weight were added to a reaction vessel, agitated under nitrogen and heated to 72°C, whereupon a catalytic amount of dibutyltindilaurate was added. The reaction mixture was heated to, and agitated at about 80°C for about 21 hours from the time the reaction mixture components were charged to the reaction vessel, and then diluted with MIBK in an amount equal to 49.0% of the initial charge weight and treated with water in an amount equal to a water ratio of 1.16. The treated mixture was agitated at about 67°C for an additional six hours.

### Example 40

A hexamethylene diisocyanate homopolymer (Desmodur N-3200) containing 22.70% NCO groups by di-N-butylamine titration method analysis, N-propyl-3,4,4,5,5,6,6,7,7,8,8,9,9,10,10,10-hexadecafluoro-2-decene-1-amine (containing about 1.4% N-perfluorooctylethyl-N-propylamine) in an amount sufficient to react with 69.7% of the total NCO group charge and dry MIBK in an amount equal to 17.5% of the total charge weight were added to a reaction vessel and agitated under nitrogen. After the resultant exotherm, the reaction mixture temperature was adjusted to about 80°C and the mixture was agitated for about 2 hours from the time the reaction mixture components were charged to the reaction vessel. Wet MIBK in an amount equal to a water ratio of 1.38 was then added and the mixture agitated at about 65°C for an additional 3.25 hours.

### Example 41

A hexamethylene diisocyanate homopolymer (Desmodur N-3200) containing 22.70% NCO groups by di-N-butylamine titration method analysis, N-propyl-3,4,4,5,5,6,6,7,7,8,8,9,9,10,10,10-hexadecafluoro-2-decen-1-amine (containing about 1.4% N-perfluorooctylethyl-N-propylamine) in an amount sufficient to react with 79.1% of the total NCO group charge, and dry MIBK in an amount equal to 21.0% of the total charge weight were added to a reaction vessel and agitated under nitrogen. After the resultant exotherm, the reaction mixture temperature was adjusted to about 80°C and the mixture was agitated for two hours from the time the reaction mixture components were charged to the reaction vessel. Wet MIBK in an amount equal to a water ratio of 2.00 was then added and the mixture agitated at about 65°C for an additional 3.5 hours.

### Example 42

A hexamethylene diisocyanate homopolymer (Desmodur N-3200) in dry MIBK solution containing 13.66% NCO groups by di-N-butyl amine titration method analysis, a perfluoroalkylethyl alcohol mixture (FA) in an amount sufficient to react with 47.6% of the NCO group charge and a monoallyl ether of a poly(oxyethylene) glycol, 550 average molecular weight, in an amount sufficient to react with 40.0% of the NCO group charge were added to a reaction vessel, agitated under nitrogen and heated to 65°C, whereupon a catalytic amount of dibutyltindilaurate was added. After the resultant exotherm, the reaction mixture was agitated at about 80°C for two hours from the time of the catalyst addition and then treated with wet MIBK in an amount equal to a water ratio of 7.47. The diluted reaction mixture was agitated at about 65°C for an additional 22 hours.

### Example 43

A hexamethylene diisocyanate homopolymer (Desmodur N-3200) in dry MIBK solution containing 13.86% NCO groups by di-N-butyl amine titration method analysis, a perfluoroalkylethyl alcohol mixture (FA) in an amount sufficient to react with 43.6% of the NCO group charge and a monomethyl ether of a poly(oxyethylene) glycol, 750 average molecular weight (MPEG 750), in an amount sufficient to react with 42.8% of the NCO group charge were added to a reaction vessel, agitated under nitrogen and heated to 63°C, whereupon a catalytic amount of dibutyltindilaurate was added. After the resultant exotherm, the reaction mixture was agitated at about 90°C for two hours from the time of the catalyst addition and then treated with wet MIBK in an amount equal to a water ratio of 9.03. The diluted reaction mixture was agitated at about 65°C for an additional two hours.

### Example 44

A hexamethylene diisocyanate homopolymer (Desmodur N-3200) in dry MIBK solution containing 13.82% NCO groups by di-N-butyl amine titration method analysis and a perfluoroalkylethyl alcohol mixture (FNA)* in an amount sufficient to react with 74.3% of the NCO group charge were added to a reaction vessel, agitated under nitrogen and heated to 61°C, whereupon a catalytic amount of dibutyltindilaurate was added. After the resultant exotherm, the reaction mixture was agitated at about 80°C for two hours from the time of the catalyst addition and then treated with wet MIBK in an amount equal to a water ratio of 1.74. The diluted reaction mixture was agitated at about 65°C for an additional two hours.
- 0% to 3%: wherein z = 6,
- 45% to 52%: wherein z = 8,
- 26% to 32%: wherein z = 10,
- 10% to 14%: wherein z = 12,
- 2% to 5%: wherein z = 14,
- 0% to 2%: wherein z = 16,
- 0% to 1%: wherein z = 18, and
- 0% to 1%: wherein z = 20.

* A perfluoroalkylethyl alcohol mixture of the formula: F(CF₂)_{z}CH₂CH₂OH wherein z is predominantly 8 and 10; in a typical mixture of which the fluoroalcohols will have the following approximate composition in relation to their F(CF₂)_{Z} radicals:

### Example 45

A hexamethylene diisocyanate homopolymer (Desmodur N-3200) in dry MIBK solution containing 13.86% NCO groups by di-N-butyl amine titration method analysis, a perfluoroalkylethyl alcohol mixture (FNA) in an amount sufficient to react with 75.0% of the NCO group charge and a monomethyl ether of a poly(oxyethylene) glycol, 350 average molecular weight,in an amount sufficient to react with 20.0% of the NCO group charge were added to a reaction vessel, agitated under nitrogen, and heated to 65°C, whereupon a catalytic amount of dibutyltindilaurate was added. After the resultant exotherm, the reaction mixture was agitated at about 80°C for two hours from the time of the catalyst addition and then treated with wet MIBK in an amount equal to a water ratio of 20.91. The diluted reaction mixture was agitated at about 65°C for an additional two hours.

### Example 46

A dry MIBK solution of meta-tetramethylxylene diisocyanate (33.88% NCO groups by di-N-butyl amine titration method analysis) and 1,1,1-tris-(hydroxymethyl)ethane in a 3/1 mole ratio was agitated under nitrogen and heated to 70°C, whereupon a catalytic amount of dibutyltindilaurate was added. After the resultant exotherm, the reaction mixture was agitated at about 80°C for 1.5 hours from the time of the catalyst addition and then cooled over 1.5 hours to 49°C, whereupon a perfluoroalkylethyl alcohol mixture (FA) in an amount sufficient to react with 70.0% of the residual NCO group charge was added. An additional catalytic amount of dibutyltindilaurate was added and the resultant mixture agitated at about 80°C for three hours from the time of the fluoroalcohol mixture addition, and then treated with wet MIBK in an amount equal to a water ratio of 1.62. The diluted reaction mixture was agitated at about 65°C for an additional 16 hours.

### Example 47

A trifunctional polyisocyanate (Mondur CB-75) derived from toluene diisocyanate and 1,1,1-tris-(hydroxymethyl)propane containing 12.56% NCO groups by di-N-butyl amine titration method analysis, a perfluoroalkylethyl alcohol mixture (FA) in an amount sufficient to react with 59.6% of the NCO group charge and dry MIBK in an amount equal to 10.8% of the total charge weight were added to a reaction vessel, agitated under nitrogen and heated to 68°C, whereupon a catalytic amount of dibutyltindilaurate was added. After the resultant exotherm, the reaction mixture was agitated at about 80°C for two hours from the time of the catalyst addition, and then treated with wet MIBK in an amount equal to a water ratio of 1.38. The diluted mixture was agitated at about 65°C for an additional four hours.

### Example 48 to 50

The procedure of Example 47 was repeated with the modifications set forth in Table 5.

**TABLE 5**

| Example | Step One | | Step Two | |
|---|---|---|---|---|
| | Rx.% | Cat.°C | Water Ratio | Time, Hrs |
| 56 | 69.7 | 60 | 1.36 | 4 |
| 57 | 80.2 | 58 | 2.23 | 4.25 |
| 58 | 89.5 | 67 | 4.52 | 4 |

### Example 51

A hexamethylene diisocyanate homopolymer (Desmodur N-3200) and a trifunctional polyisocyanate (Mondur CB-75) derived from toluene diisocyanate and 1,1,1-tris(hydroxymethyl)propane containing 22.71% and 12.56% NCO groups by di-N-butylamine titration method analysis, respectively, were charged to a reaction vessel in relative amounts that the hexamethylene diisocyanate homopolymer contributed 75.0% of the total NCO group charge, along with a perfluoroalkylethyl alcohol (FA) in an amount sufficient to react with 69.9% of the total NCO group charge, and dry MIBK in amount equal to 21.9% of the total charge weight. The mixture was agitated under nitrogen and heated to 64°C, whereupon a catalytic amount of dibutyltindilaurate was added. After the resultant exotherm, the reaction mixture was agitated at about 80°C for two hours from the time of the catalyst addition and then treated with wet MIBK in an amount equal to a water ratio of 1.32. The diluted reaction mixture was agitated at about 65°C for two additional hours.

### Example 52

The procedure of Example 51 was repeated except that the perfluoroalkylethyl alcohol was charged in an amount sufficient to react with 80.2% of the total NCO group charge.

### Example 53

A hexamethylene diisocyanate homopolymer (Desmodur N-3200) in dry MIBK solution containing 13.69% NCO groups by di-N-butyl amine titration method analysis, a perfluoroalkylethyl alcohol mixture (FA) in an amount sufficient to react with 40.0% of the NCO group charge and a monomethyl ether of a poly(oxyethylene) glycol, 750 average molecular weight (MPEG 750), in an amount sufficient to react with 40.0% of the NCO group charge were added to a reaction vessel, agitated under nitrogen and heated to 60°C, whereupon a catalytic amount of dibutyltindilaurate was added. After the resultant exotherm, the reaction mixture was agitated at about 90°C for two hours from the time of the catalyst addition and then treated with wet MIBK in an amount equal to a water ratio of 2.48. The diluted reaction mixture was agitated at about 65°C for an additional two hours.

## Claims

1. Use in the treatment of a fibre or fabric of a water dispersion or organic solvent solution of a polyfluoro organic compound to impart water, oil and soil repellency and/or soil release properties to said fabric, said polyfluoro organic compound comprising a compound having at least one urea linkage which compound is the reaction product of (1) an excess of at least one polyisocyanate containing at least three isocyanate groups, with (2) at least one fluorochemical compound which contains per molecule (a) a single functional group having one or more Zerewitinoff hydrogen atoms and (b) at least two carbon atoms each of which contains at least two fluorine atoms, and then with (3) water in an amount sufficient to react with the residual isocyanate groups, from 5% to 60% of the isocyanate groups in said polyisocyanate.

2. Use according to claim 1 wherein the amount of water is sufficient to react with 10% to 35% of said isocyanate groups.

3. Use according to claim 1 wherein the amount of water is sufficient to react with 15% to 30% of said isocyanate group.

4. Use according to any one of the preceding claims wherein said fluorochemical compound which contains a single functional group can be represented by the formula:
R^{f}-Rₖ-X-H
in which
R^{f} is a monovalent aliphatic group containing at least two carbon atoms each of which contains two fluorine atoms;
R is a divalent organic radical;
k is 0 or 1; and
X is -0-, -S-, or -N(R¹)- in which R¹ is H, alkyl containing 1 to 6 carbon atoms or a R^{f}-Rₖ group.

5. Use according to any one of claims 1 to 3 wherein said fluorochemical compound which contains a single functional group can be represented by the formula:
R^{f}-Rₖ-R²-X-H
wherein
R is the divalent radical -CₘH₂ₘSO-, -CₘH₂ₘSO₂-, -SO₂N(R³)-, or -CON(R³)- in which m is 1 to 22 and R³ is H or alkyl of 1 to 6 carbon atoms;
R² is the divalent linear hydrocarbon radical -CₙH₂ₙ- which can be optionally end-capped by 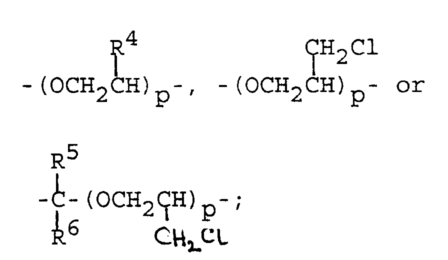
n is 0 to 12, p is 1 to 50, and R⁴, R⁵ and R⁶ are the same or different H or alkyl containing 1 to 6 carbon atoms;
X is -O-, -S-, or -N(R⁷)- in which R⁷ is H, alkyl containing 1 to 6 carbon atoms or a R^{f}-Rₖ-R²- group;
Rf is a monovalent aliphatic group containing at least two carbon atoms each of which contains two fluorine atoms; and
k is 0 or 1

6. Use according to claim 5 wherein R^{f} is a fully-fluorinated straight or branched aliphatic radical of 3 to 20 carbon atoms which can be interrupted by oxygen atoms.

7. Use according to claim 6 wherein said fluorochemical compound which contains a single functional group can be represented by the formula:
R^{f}-(CH₂)_{q}-X-H
in which
R^{f} is the perfluoroalkyl group CF₃CF₂(CF₂)ᵣ in which r is 2 to 18; and
q is 1, 2 or 3.

8. Use according to claim 7 of a mixture of fluorochemical compounds wherein
R^{f} is the perfluoroalkyl group CF₃CF₂(CF₂)ᵣ; and r is 2, 4, 6, 8, 10, 12, 14, 16 and 18, and such compounds differ by the value of r.

9. Use according to claim 8 wherein X is oxygen and q is 2.

10. Use according to claim 8 wherein r is predominantly 4, 6 and 8.

11. Use according to claim 8 wherein r is predominantly 6 and 8.

12. Use according to claim 8 wherein X is -N(R⁷)-, R⁷ is H and q is 2.

13. Use according to claim 4 wherein said fluorochemical compound which contains a single functional group can be represented by the formula: H(CF₂CF₂)_{w}CH₂OH in which w is 1-10.

14. Use according to claim 4 wherein said fluorochemical compound which contains a single functional group can be represented by the formula: CF₃(CF₃)CHOH.

15. Use according to any one of the preceding claims wherein between 1% and 60% of said isocyanate groups are reacted with at least one non-fluorinated organic compound which contains a single functional group.

16. Use according to claim 15 wherein said non-fluorinated organic compound can be represented by the formula:
R¹⁰-R¹¹ₖ-YH
wherein
R¹⁰ is a C₁-C₁₈ alkyl, a C₁-C₁₈ omega-alkenyl radical or a C₁-C₁₈ omega-alkenoyl;
R¹¹ is 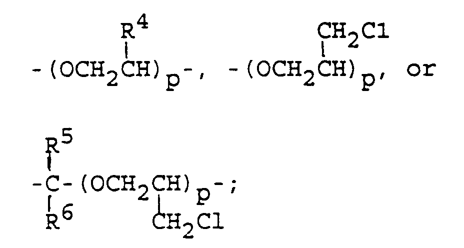
R⁴, R⁵ and R⁶ are the same or different H or alkyl radical containing 1 to 6 carbon atoms and p is 1 to 50;
k is 0 or 1;
Y is -O-, -S-, or -N(R⁷)- in which R⁷ is H or alkyl containing 1 to 6 carbon atoms.

17. Use according to claim 16 wherein said non-fluorinated compound is an alkanol.

18. Use according to claim 16 wherein said non-fluorinated compound is a monoalkyl ether of a poly(oxyalkylene)glycol.

19. Use according to any one of claims 1 to 3 wherein said polyisocyanate can be represented by the formula: 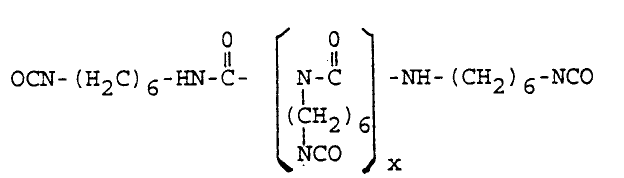 wherein x is an integer equal to or greater than 1.

20. Use according to claim 19 wherein x is 1 to 8.

21. Use according to any one of claims 1 to 3 wherein said polyisocyanate can be represented by the formula: 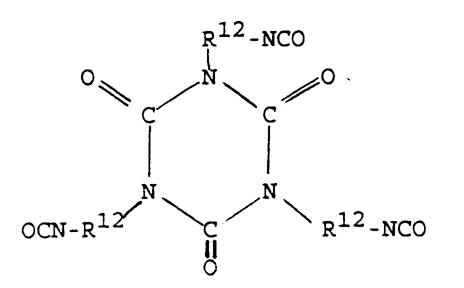 wherein R¹² is a divalent hydrocarbon group.

22. Use according to any one of claims 1 to 3 wherein said polyisocyanate can be represented by the formula: 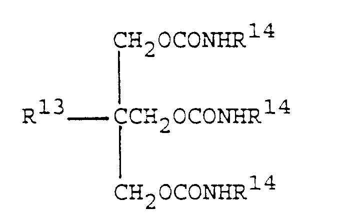 wherein R¹³ is methyl or ethyl, and
R¹⁴ is

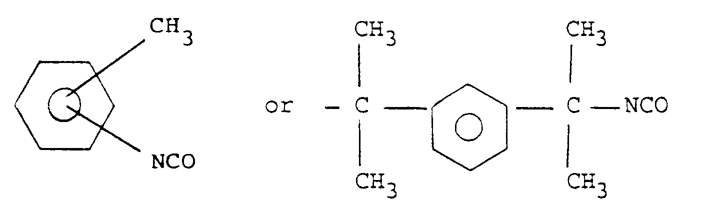

23. A polyfluoro organic compound comprising a compound having at least one urea linkage which compound is the reaction product of (1) an excess of at least one polyisocyanate, with (2) at least one fluorochemical compound which contains per molecule (a) a single functional group having one or more Zerewitinoff hydrogen atoms and (b) at least two carbon atoms each of which contains at least two fluorine atoms, and then with (3) water, the water being used in an in an amount sufficient to react with all residual isocyanate groups, said residual groups constituting from 5% to 60% of the isocyanate groups in said polyisocyanate, wherein said polyisocyanate contains at least three isocyanate groups.

24. A compound as claimed in claim 23 wherein said polyisocyanate comprises a compound of the general formula:- 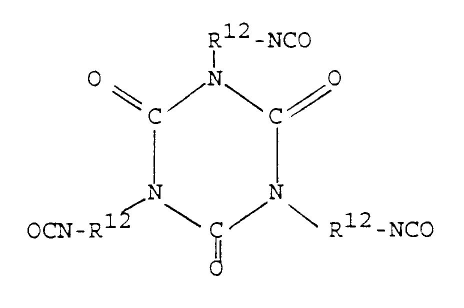 wherein R¹² is a divalent hydrocarbon group.

25. A compound as claimed in claim 24 in which R¹² is hexamethylene, tolylene or cyclohexylene.

26. A compound as claimed in claim 23 wherein said polyisocyanate comprises a compound of the general formula:- 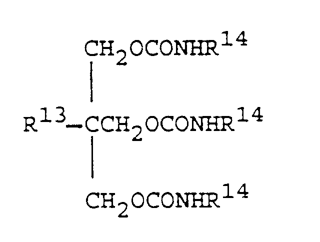 wherein R¹³ is methyl or ethyl, and
R¹⁴ is

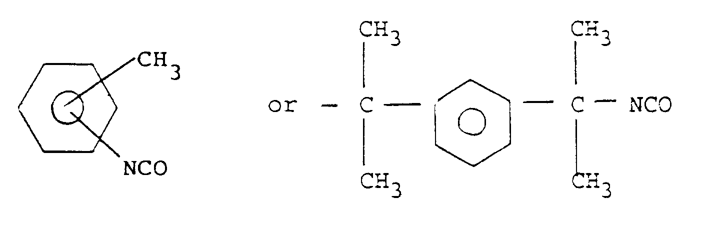

27. A process for imparting oil, water- and soil-repellency and/or soil release properties to a fabric or fibre substrate which comprises applying to the substrate a polyfluoro organic compound of any one of claims 23 to 26.

28. A textile substrate containing, as a water-, oil- or soil-repellent or soil-release agent, a polyfluoro organic compound having at least one urea linkage which compound is derived from (A) at least one polyisocyanate which contains at least three isocyanate groups per molecule, (B) at least one fluorochemical compound which contains per molecule (i) a single functional group having one or more Zerewitinoff hydrogen atoms and (ii) at least two carbon atoms each of which contains at least two fluorine atoms, and (C) water in an amount sufficient to react with from 5% to 60% of the isocyanate groups in said polyisocyanate.

29. A process for the preparation of a polyfluoro organic compound having at least one urea linkage which comprises (I) reacting (a) at least one polyisocyanate or mixture of polyisocyanates at least one of which contains at least three isocyanate groups per molecule with (b) a stoichiometric deficiency of at least one fluorochemical compound which contains per molecule (i) a single functional group having one or more Zerewitinoff hydrogen atoms and (ii) at least two carbon atoms each of which contains at least two fluorine atoms, and (II) thereafter forming one or more urea linkages per molecule of said polyisocyanate by reacting the remaining isocyanate groups with water in an amount sufficient to react with from 5% to 60% of the isocyanate groups in said polyisocyanate.

## Patentansprüche

1. Verwendung bei der Behandlung einer Faser oder eines Stoffes einer wäßrigen Dispersion oder einer organischen Lösungsmittel-Lösung einer polyfluororganischen Verbindung, um dem Stoff Wasser-, Öl- und Schmutzabweisung und/oder Soil-Release-Eigenschaften zu verleihen, wobei die polyfluororganische Verbindung eine Verbindung umfaßt, die wenigstens eine Harnstoffbindung aufweist, wobei die Verbindung das Reaktionsprodukt ist von (1) einem Überschuß von wenigstens einem Polyisocyanat, das wenigstens 3 Isocyanatgruppen enthält, mit (2) wenigstens einer fluorchemischen Verbindung, die pro Molekül (a) eine einzige funktionelle Gruppe, die ein oder mehrere Zerewitinoff-Wasserstoffatome aufweist, und (b) wenigstens zwei Kohlenstoffatome enthält, von denen jedes wenigstens 2 Fluoratome enthält, und sodann mit (3) Wasser in einer Menge, die ausreicht, um die restlichen Isocyanatgruppen von 5 % bis 60 % der Isocyanatgruppen in dem Polyisocyanat umzusetzen.

2. Verwendung nach Anspruch 1, bei der die Menge an Wasser ausreicht, um mit 10 % bis 35 % der Isocyanatgruppen zu reagieren.

3. Verwendung nach Anspruch 1, bei der die Menge an Wasser ausreicht, um mit 15 % bis 30 % der Isocyanatgruppe zu reagieren.

4. Verwendung nach einem der vorhergehenden Ansprüche, bei der die fluorchemische Verbindung, die eine einzige funktionelle Gruppe enthält, durch die Formel:
R^{f}-Rₖ-X-H
dargestellt werden kann, in der
R^{f} für eine einwertige aliphatische Gruppe steht, die wenigstens 2 Kohlenstoffatome enthält, die jeweils 2 Fluoratome enthalten;
R für einen zweiwertigen organischen Rest steht;
k für 0 oder 1 steht; und
X für -O-, -S- oder -N(R¹)- steht, worin R¹ für H, Alkyl, das 1 bis 6 Kohlenstoffatome enthält, oder eine R^{f}-Rₖ-Gruppe steht.

5. Verwendung nach einem der Anrüche 1 bis 3, bei der die fluorchemische Verbindung, die eine einzige funktionelle Gruppe enthält, durch die Formel
R^{f}-Rₖ-R²-X-H
dargestellt werden kann, worin
R für den zweiwertigen Rest -CₘH₂ₘSO-, -CₘH₂ₘSO₂-, -SO₂N(R³)- oder -CON(R³)- steht, in dem m für eine Zahl von 1 bis 22 steht und R³ für H oder Alkyl mit 1 bis 6 Kohlenstoffatomen steht;
R² für den zweiwertigen linearen Kohlenwasserstoffrest -CₙH₂ₙ- steht, der gegebenenfalls endabgeschlossen sein kann mit 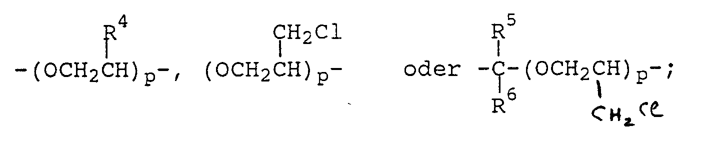
n für eine Zahl von 0 bis 12 steht, p für eine Zahl von 1 bis 50 steht und R⁴, R⁵ und R⁶ gleich oder verschieden für H oder Alkyl stehen, das 1 bis 6 Kohlenstoffatome enthält;
X für -O-, -S- oder -N(R⁷)- steht, worin R⁷ für H, Alkyl, das 1 bis 6 Kohlenstoffatome enthält, oder eine R^{f}-Rₖ-R²-Gruppe steht;
R^{f} für eine einwertige aliphatische Gruppe steht, die wenigstens zwei Kohlenstoffatome enthält, von denen jedes zwei Fluoratome enthält; und
k 0 oder 1 bedeutet.

6. Verwendung nach Anspruch 5, bei der R^{f} für einen vollständig fluorierten geradkettigen oder verzweigten aliphatischen Rest von 3 bis 20 Kohlenstoffatomen steht, der durch Sauerstoffatome unterbrochen sein kann.

7. Verwendung nach Anspruch 6, bei der die fluorchemische Verbindung, die eine einzige funktionelle Gruppe enthält, durch die Formel:
R^{f}-(CH₂)_{q}-X-H
dargestellt werden kann, in der
R^{f} für die Perfluoralkylgruppe CF₃CF₂(CF₂)ᵣ steht, in der r eine Zahl von 2 bis 18 bedeutet; und
q 1, 2 oder 3 bedeutet.

8. Verwendung nach Anspruch 7 eines Gemisches von fluorchemischen Verbindungen, worin
R^{f} für die Perfluoralkylgruppe CF₃CF₂(CF₂)ᵣ steht und r für 2, 4, 6, 8, 10, 12, 14, 16 und 18 steht und solche Verbindungen sich um den Wert von r unterscheiden.

9. Verwendung nach Anspruch 8, bei der X für Sauerstoff steht und q für 2 steht.

10. Verwendung nach Anspruch 8, bei der r vorwiegend für 4, 6 und 8 steht.

11. Verwendung nach Anspruch 8, bei der r vorwiegend für 6 und 8 steht.

12. Verwendung nach Anspruch 8, bei der X für -N(R⁷)- steht; R⁷ für H steht und q für 2 steht.

13. Verwendung nach Anspruch 4, bei der die fluorchemische Verbindung, die eine einzige funktionelle Gruppe enthält, durch die Formel: H(CF₂CF₂)_{w}CH₂OH dargestellt werden kann, worin w für 1-10 steht.

14. Verwendung nach Anspruch 4, worin die fluorchemische Verbindung, die eine einzige funktionelle Gruppe enthält, durch die Formel: CF₃(CF₃)CHOH dargestellt werden kann.

15. Verwendung nach einem der vorhergehenden Ansprüche, bei der zwischen 1 % und 60 % der Isocyanatgruppen mit wenigstens einer nichtfluorierten organischen Verbindung umgesetzt werden, die eine einzige funktionelle Gruppe enthält.

16. Verwendung nach Anspruch 15, bei der die nichtfluorierte organische Verbindung kann durch die Formel
R¹⁰-R¹¹ₖ-YH
dargestellt werden, worin
R¹⁰ für ein C₁-C₁₈-Alkyl, einen C₁-C₁₈-omega-Alkenylrest oder ein C₁-C₁₈-omega-Alkenoyl steht;
R¹¹ für 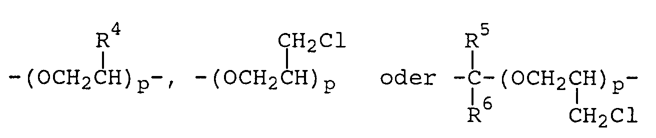 steht;
R⁴, R⁵ und R⁶ gleich oder verschieden für H oder einen Alkylrest stehen, der 1 bis 6 Kohlenstoffatome enthält, und p für eine Zahl von 1 bis 50 steht;
k für 0 oder 1 steht;
Y für -O-, -S- oder -N(R⁷)- steht, worin R⁷ für H oder Alkyl steht, das 1 bis 6 Kohlenstoffatome enthält.

17. Verwendung nach Anspruch 16, bei der die nichtfluorierte Verbindung ein Alkanol ist.

18. Verwendung nach Anspruch 16, bei der die nichtfluorierte Verbindung für einen Monoalkylether eines Poly(oxyalkylen)-glycols steht.

19. Verwendung nach einem der Ansprüche 1 bis 3, bei der das Polyisocyanat durch die Formel: 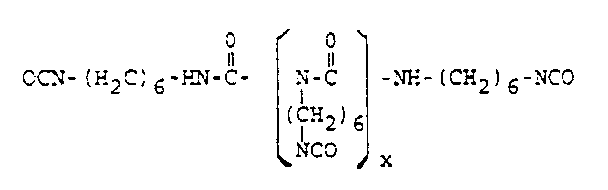 dargestellt werden kann, worin x eine ganze Zahl bedeutet, die größer oder gleich 1 ist.

20. Verwendung nach Anspruch 19, bei der x eine Zahl von 1 bis 8 bedeutet.

21. Verwendung nach einem der Ansprüche 1 bis 3, bei der das Polyisocyanat durch die Formel: 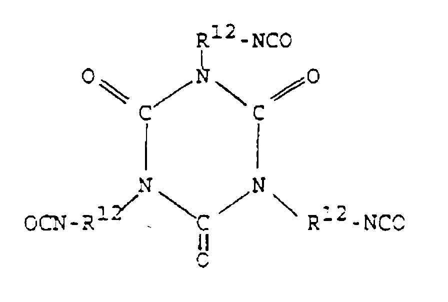 dargestellt werden kann, worin R¹² eine zweiwertige Kohlenwasserstoffgruppe bedeutet.

22. Verwendung nach einem der Ansprüche 1 bis 3, worin das Polyisocyanat durch die Formel: 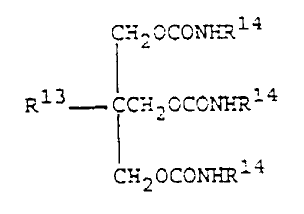 dargestellt werden kann, worin R¹³ für Methyl oder Ethyl steht und
R¹⁴ für 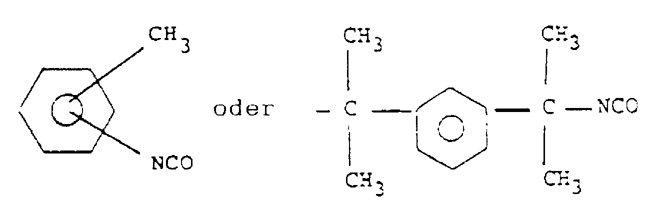 steht.

23. Polyfluororganische Verbindung, umfassend eine Verbindung, die wenigstens eine Harnstoffbindung aufweist, wobei die Verbindung das Reaktionsprodukt ist von (1) einem Überschuß von wenigstens einem Polyisocyanat mit (2) wenigstens einer fluorchemischen Verbindung, die pro Molekül (a) eine einzige funktionelle Gruppe, die ein oder mehrere Zerewitinoff-Wasserstoffatome enthält, und (b) wenigstens zwei Kohlenstoffatome, die jeweils wenigstens 2 Fluoratome enthalten, enthält, und dann mit (3) Wasser, wobei das Wasser in einer Menge verwendet wird, die ausreicht, um mit sämtlichen restlichen Isocyanatgruppen zu reagieren, wobei diese restlichen Gruppen 5 % bis 60 % der Isocyanatgruppen in dem Polyisocyanat ausmachen, worin das Polyisocyanat wenigstens drei Isocyanatgruppen enthält.

24. Verbindung nach Anspruch 23, worin das Polyisocyanat eine Verbindung der allgemeinen Formel: 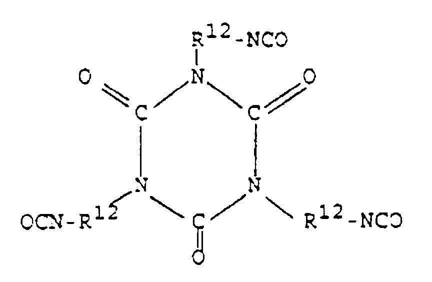 umfaßt, worin R¹² für eine zweiwertige Kohlenwasserstoffgruppe steht.

25. Verbindung nach Anspruch 24, worin R¹² für Hexamethylen, Tolylen oder Cyclohexylen steht.

26. Verbindung nach Anspruch 23, worin das Polyisocyanat eine Verbindung der allgemeinen Formel: 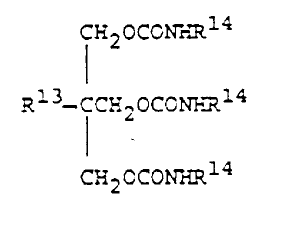 umfaßt, worin R¹³ für Methyl oder Ethyl steht und R¹⁴ für 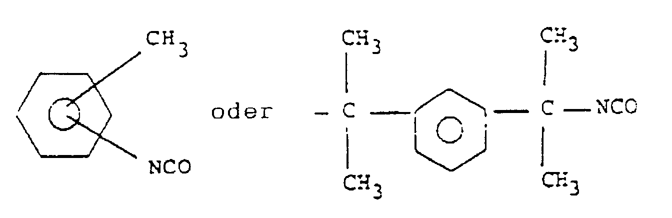 steht.

27. Verfahren zur Verleihung von Öl-, Wasser- und Schmutzabweisung und/oder Soil-Release-Eigenschaften an einen Stoff oder an ein Fasersubstrat, welches das Aufbringen einer polyfluororganischen Verbindung nach einem der Ansprüche 23 bis 26 auf das Substrat umfaßt.

28. Textilsubstrat, das als wasser-, öl- oder schmutzabweisendes oder Soil-Release-Mittel, eine polyfluororganische Verbindung enthält, die wenigstens eine Harnstoffbindung aufweist, wobei sich diese Verbindung ableitet von (A) wenigstens einem Polyisocyanat, das wenigstens 3 Isocyanatgruppen pro Molekül enthält, (B) wenigstens einer fluorchemischen Verbindung, die pro Molekül (i) eine einzige funktionelle Gruppe, die ein oder mehrere Zerewitinoff-Wasserstoffatome aufweist, und (ii) wenigstens zwei Kohlenstoffatome, die jeweils wenigstens 2 Fluoratome enthalten, enthält, und (C) Wasser in einer Menge, die ausreicht, um mit 5 % bis 60 % der Isocyanatgruppen in dem Polyisocyanat zu reagieren.

29. Verfahren zur Herstellung einer polyfluororganischen Verbindung, die wenigstens eine Harnstoffbindung aufweist, welches umfaßt (I) Umsetzung von (a) wenigstens einem Polyisocyanat oder einem Gemisch von Polyisocyanaten, wovon wenigstens eines wenigstens drei Isocyanatgruppen pro Molekül enthält, mit (b) einem stöchiometrischen Unterschuß von wenigstens einer fluorchemischen Verbindung, welche pro Molekül (i) eine einzige funktionelle Gruppe, die ein oder mehrere Zerewitinoff-Wasserstoffatome aufweist, und (ii) wenigstens zwei Kohlenstoffatome, die jeweils wenigstens zwei Fluoratome enthalten, enthält und (II) anschließendes Bilden von einer oder mehreren Harnstoffbindungen pro Molekül Polyisocyanat durch Umsetzung der restlichen Isocyanatgruppen mit Wasser in einer Menge, die ausreicht, um mit 5 % bis 60 % der Isocyanatgruppen in dem Polyisocyanat zu reagieren.

## Revendications

1. Utilisation, dans le traitement d'une fibre ou d'une étoffe, d'une dispersion aqueuse ou d'une solution dans un solvant organique d'un composé organique polyfluoré pour conférer à ladite étoffe des propriétés de répulsion d'eau, d'huile et des salissures et/ou de décollement des salissures, ledit composé organique polyfluoré comprenant un composé ayant au moins une liaison urée, lequel composé est le produit réactionnel de (1) un excès d'au moins un polyisocyanate contenant au moins trois groupes isocyanate, avec (2) au moins un composé chimique fluoré qui contient, par molécule, (a) un seul groupe fonctionnel ayant un ou plusieurs atomes d'hydrogène de Zerewitinoff et (b) au moins deux atomes de carbone qui portent chacun au moins deux atomes de fluor, puis avec (3) de l'eau en une quantité suffisante pour réagir avec les groupes isocyanate résiduels, représentant 5 % à 60 % des groupes isocyanate dudit polyisocyanate.

2. Utilisation selon la revendication 1, dans laquelle la quantité d'eau est suffisante pour réagir avec 10 % à 35 % desdits groupes isocyanate.

3. Utilisation selon la revendication 1, dans laquelle la quantité d'eau est suffisante pour réagir avec 15 % à 30 % desdits groupes isocyanate.

4. Utilisation selon l'une quelconque des revendications précédentes, dans laquelle ledit composé chimique fluoré qui contient un seul groupe fonctionnel peut être représenté par la formule :
R^{f}-Rₖ-X-H
dans laquelle
R^{f} est un groupe aliphatique monovalent contenant au moins deux atomes de carbone qui portent chacun deux atomes de fluor ;
R est un radical organique divalent ;
k est 0 ou 1 ; et
X est -O-, -S- ou -N(R¹)-, où R¹ est H, un groupe alkyle contenant 1 à 6 atomes de carbone ou un groupe R^{f}-Rₖ.

5. Utilisation selon l'une quelconque des revendications 1 à 3, dans laquelle ledit composé chimique fluoré qui contient un seul groupe fonctionnel peut être représenté par la formule :
R^{f}-Rₖ-R²-X-H
dans laquelle
R est le radical divalent -CₘH₂ₘSO-, -CₘH₂ₘSO₂-, -SO₂N(R³)- ou -CON(R³)- où m est de 1 à 22 et R³ est H ou un groupe alkyle de 1 à 6 atomes de carbone ;
R² est le radical hydrocarboné linéaire divalent -CₙH₂ₙ- qui peut être facultativement terminé par 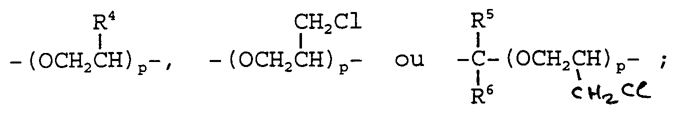
n est de 0 à 12, p est de 1 à 50, et R⁴, R⁵ et R⁶ sont identiques ou différents et chacun est H ou un groupe alkyle contenant 1 à 6 atomes de carbone ;
X est -O-, -S- ou -N(R⁷)- où R⁷ est H, un groupe alkyle contenant 1 à 6 atomes de carbone ou un groupe R^{f}-Rₖ-R²- ;
R^{f} est un groupe aliphatique monovalent contenant au moins deux atomes de carbone qui portent chacun deux atomes de fluor ; et
k est 0 ou 1.

6. Utilisation selon la revendication 5, dans laquelle R^{f} est un radical aliphatique linéaire ou ramifié entièrement fluoré de 3 à 20 atomes de carbone qui peut être interrompu par des atomes d'oxygène.

7. Utilisation selon la revendication 6, dans laquelle ledit composé chimique fluoré qui contient un seul groupe fonctionnel peut être représenté par la formule :
R^{f}-(CH₂)_{q}-X-H
dans laquelle
R^{f} est le groupe perfluoroalkyle CF₃CF₂(CF₂)ᵣ où r est de 2 à 18 ; et
q est 1, 2 ou 3.

8. Utilisation selon la revendication 7 d'un mélange de composés chimiques fluorés dans lesquels
R^{f} est le groupe perfluoralkyle CF₃CF₂(CF₂)ᵣ ; et r est 2, 4, 6, 8, 10, 12, 14, 16 et 18, et ces composés diffèrent par la valeur de r.

9. Utilisation selon la revendication 8, dans laquelle X est l'oxygène et q est 2.

10. Utilisation selon la revendication 8, dans laquelle r est de façon prédominante 4, 6 et 8.

11. Utilisation selon la revendication 8, dans laquelle r est de façon prédominante 6 et 8.

12. Utilisation selon la revendication 8, dans laquelle X est -N(R⁷)-, R⁷ est H et q est 2.

13. Utilisation selon la revendication 4, dans laquelle ledit composé chimique fluoré qui contient un seul groupe fonctionnel peut être représenté par la formule : H(CF₂CF₂)_{w}CH₂OH dans laquelle w est de 1 à 10.

14. Utilisation selon la revendication 4, dans laquelle ledit composé chimique fluoré qui contient un seul groupe fonctionnel peut être représenté par la formule : CF₃(CF₃)CHOH.

15. Utilisation selon l'une quelconque des revendications précédentes, dans laquelle 1 % à 60 % desdits groupes isocyanate ont réagi avec au moins un composé organique non fluoré qui contient un seul groupe fonctionnel.

16. Utilisation selon la revendication 15, dans laquelle ledit composé organique non fluoré peut être représenté par la formule :
R¹⁰-R¹¹ₖ-YH
dans laquelle
R¹⁰ est un groupe alkyle en C₁-C₁₈, un groupe oméga-alcényle en C₁-C₁₈ ou un groupe oméga-alcénoyle en C₁-C₁₈ ;
R¹¹ est 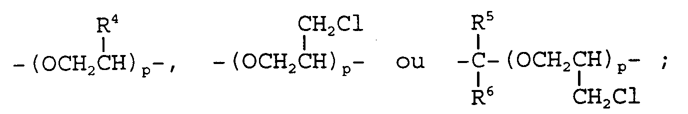
R⁴, R⁵ et R⁶ sont identiques ou différents et chacun est H ou un groupe alkyle contenant 1 à 6 atomes de carbone et p est de 1 à 50 ;
k est 0 ou 1 ;
Y est -O-, -S- ou -N(R⁷)- où R⁷ est H ou un groupe alkyle contenant 1 à 6 atomes de carbone.

17. Utilisation selon la revendication 16, dans laquelle ledit composé non fluoré est un alcanol.

18. Utilisation selon la revendication 16, dans laquelle ledit composé non fluoré est un éther monoalkylique d'un poly(oxyalkylène)glycol.

19. Utilisation selon l'une quelconque des revendications 1 à 3, dans laquelle ledit polyisocyanate peut être représenté par la formule : 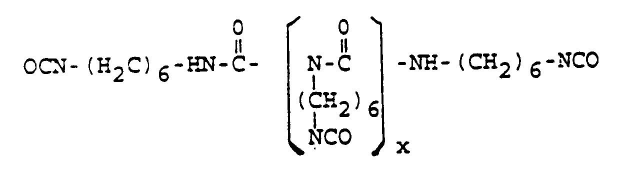 dans laquelle x est un nombre entier égal ou supérieur à 1.

20. Utilisation selon la revendication 19, dans laquelle x est de 1 à 8.

21. Utilisation selon l'une quelconque des revendications 1 à 3, dans laquelle ledit polyisocyanate peut être représenté par la formule : 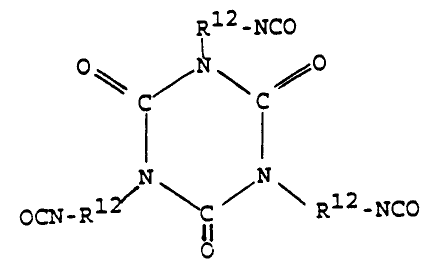 dans laquelle R¹² est un groupe hydrocarboné divalent.

22. Utilisation selon l'une quelconque des revendications 1 à 3, dans laquelle ledit polyisocyanate peut être représenté par la formule : 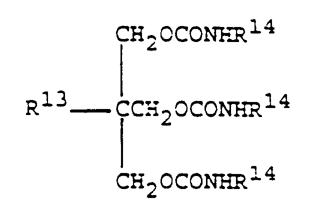 dans laquelle
R¹³ est un groupe méthyle ou éthyle, et
R¹⁴ est

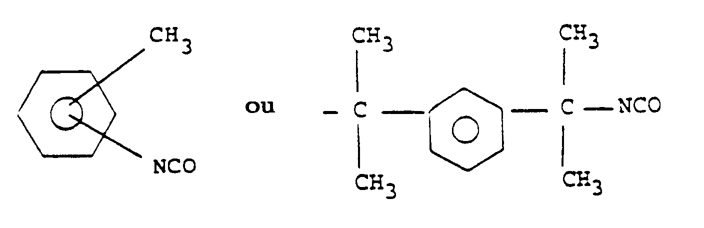

23. Un composé organique polyfluoré comprenant un composé ayant au moins une liaison urée, lequel composé est le produit réactionnel de (1) un excès d'au moins un polyisocyanate, avec (2) au moins un composé chimique fluoré qui contient, par molécule, (a) un seul groupe fonctionnel ayant un ou plusieurs atomes d'hydrogène de Zerewitinoff et (b) au moins deux atomes de carbone qui portent chacun au moins deux atomes de fluor, puis avec (3) de l'eau, l'eau étant utilisée en une quantité suffisante pour réagir avec tous les groupes isocyanate résiduels, lesdits groupes résiduels représentant 5 % à 60 % des groupes isocyanate dudit polyisocyanate, dans lequel ledit polyisocyanate contient au moins trois groupes isocyanate.

24. Un composé tel que revendiqué dans la revendication 23, dans lequel ledit polyisocyanate comprend un composé de la formule générale : 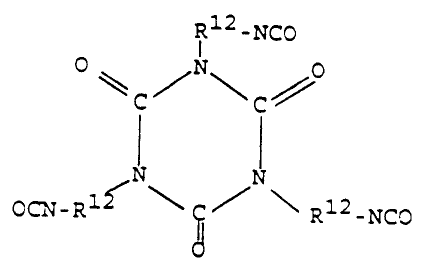 dans laquelle R¹² est un groupe hydrocarboné divalent.

25. Un composé tel que revendiqué dans la revendication 24, dans lequel R¹² est un groupe hexaméthylène, tolylène ou cyclohexylène.

26. Un composé tel que revendiqué dans la revendication 23, dans lequel ledit polyisocyanate comprend un composé de la formule générale : 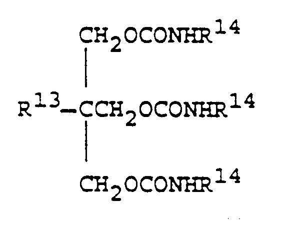 dans laquelle
R¹³ est un groupe méthyle ou éthyle, et
R¹⁴ est

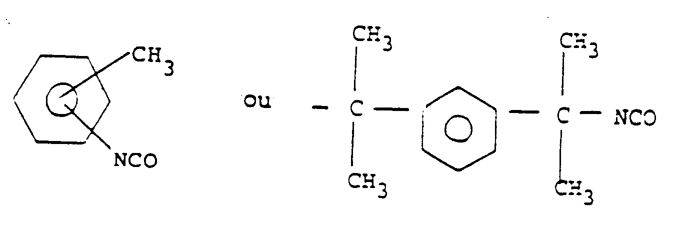

27. Un procédé pour conférer des propriétés de répulsion d'huile, d'eau et des salissures et/ou de décollement des salissures à un substrat qui est une étoffe ou une fibre, qui consiste à appliquer au substrat un composé organique polyfluoré de l'une quelconque des revendications 23 à 26.

28. Un substrat textile contenant, à titre d'agent de répulsion d'eau, d'huile ou des salissures ou de décollement des salissures, un composé organique polyfluoré ayant au moins une liaison urée, lequel composé est dérivé de (A) au moins un polyisocyanate qui contient au moins trois groupes isocyanate par molécule, (B) au moins un composé chimique fluoré qui contient, par molécule, (i) un seul groupe fonctionnel ayant un ou plusieurs atomes d'hydrogène de Zerewitinoff et (ii) au moins deux atomes de carbone qui portent chacun au moins deux atomes de fluor, et (C) de l'eau en une quantité suffisante pour réagir avec 5 % à 60 % des groupes isocyanate dudit polyisocyanate.

29. Un procédé pour la préparation d'un composé organique polyfluoré ayant au moins une liaison urée, qui consiste à (I) faire réagir (a) au moins un polyisocyanate ou un mélange de polyisocyanates dont au moins l'un contient au moins trois groupes isocyanate par molécule avec (b) un déficit stoechiométrique d'au moins un composé chimique fluoré qui contient, par molécule, (i) un seul groupe fonctionnel ayant un ou plusieurs atomes d'hydrogène de Zerewitinoff et (ii) au moins deux atomes de carbone qui portent chacun au moins deux atomes de fluor, puis (II) former une ou plusieurs liaisons urée par molécule dudit polyisocyanate en faisant réagir les groupes isocyanate restants avec de l'eau en une quantité suffisante pour réagir avec 5 % à 60 % des groupes isocyanate dudit polyisocyanate.
